Europäisches Patentamt

(19) European Patent Office

Office européen des brevets

(11) Publication number : **0 364 839 B1**

(12) # EUROPEAN PATENT SPECIFICATION

(45) Date of publication of patent specification :
05.06.91 Bulletin 91/23

(51) Int. Cl.⁵ : **A61M 5/32**

(21) Application number : **89118647.0**

(22) Date of filing : **06.10.89**

(54) Protected needle syringe.

(30) Priority : **18.10.88 IT 2234588**

(43) Date of publication of application :
**25.04.90 Bulletin 90/17**

(45) Publication of the grant of the patent :
**05.06.91 Bulletin 91/23**

(84) Designated Contracting States :
**AT BE CH DE ES FR GB GR IT LI NL SE**

(56) References cited :
**EP-A- 0 047 442**
**GB-A- 2 198 353**
**US-A- 4 747 835**

(73) Proprietor : **GI.BI.EFFE. S.R.L.**
**Via Aurelio Saffi 21**
**I-20123 Milan (IT)**

(72) Inventor : **Lo Duca, Carmelo**
**Via Val Di Sole, 11**
**I-20141 Milan (IT)**

(74) Representative : **Giambrocono, Alfonso, Dr.**
**Ing. et al**
**Ing. A. Giambrocono & C. S.r.l. Via Rosolino**
**Pilo 19/B**
**I-20129 Milano (IT)**

## Description

The present invention concerns a syringe for medical use, and more precisely a disposable syringe with protected needle.

It is known that the syringes of said type essentially comprise a hollow body, said body having a cylindrical shape with one end open, the other end being tapered and having such a shape as to receive a normal needle for injections. Within the cylindrical body a tight sealing piston can be moved in both directions, said pistons being driven by means of a stem that projects out of said open end of the cylindrical body.

At the moment of sale, the syringe needle is normally protected by means of a cap to be removed before using the syringe.

These syringes have the cylindrical body, stem and cap made of a substantially stiff synthetic material, the piston being on the contrary made of rubber or a resilient synthetic material having a suitable hardness. The syringe is employed in the following known way.

After removing the needle protection cap by grasping with the fingers the tip of the stem, the piston is shifted away from its position by the needle, or terminal position, in which the piston normally is at the moment the syringe is purchased. This displacement causes a depression to arise inside the syringe, by means of which, from a vial or ampoule, the proper amount of liquid drug is sucked through the needle.

Thereafter, the injection is performed in the usual known way. The needle protection cap is finally put on again before disposing of the whole assembly.

The low-cost syringes made of synthetic material are normally used only once, and then disposed off. This is due to obvious hygienic reasons that have become even more stringent, nowadays, mainly due to the diffusion of AIDS (aquired immuno-deficiency syndrome), one of the best transmission vehicles for which is indeed the used syringe.

It is however evident, that the protection cap can be easily removed, for example by children that might find said used syringes, or can be pulled-out accidentally during the transportation of waste into which the syringes are normally thrown with the serious troubles this can cause.

It is therefore highly desirable to eliminate the above-mentioned dangers by creating a syringe that, after it has been used, allows the risk of being accidentally stung for a person that imprudently or unconsciously touches said used syringes to be avoided.

In order to overcome the above mentioned problems, the prior art teaches to use caps suitable to be safely blocked on the syringe needles, so that the needles can no more be disconnected from the relevant caps after they have been forced into them.

The person handling the syringe could however have his fingers punctured while he is introducing the needle into the cap after the syringe has been used.

In order to avoid or minimize such risk, the prior art discloses to provide the caps with enlarged shields projecting substantially radially outwardly at their open end, whereby in use the caps can be hand held and the needles inserted into them with low risk of the syringe user incurring a self inflicted puncture whilst holding the cap because the user's hand is partially protected by the shields.

Such a structure has proved to present serious drawbacks because the user's hand is effectively protected only if the shield is of a large size : in such a case, however, the cap provided with large shield cannot be practically used just in consequence of its size which makes it unsuitable for packaging, storing and transportation.

Disposable safety needle caps having the hereabove mentioned features are well known and are disclosed, for example, in US-A-4 610 667, in US-A- 4 747 835, in GB-A-2 162 428 and in GB-A-2 198 353.

The main oject of the present invention is therefore to provide a needle safety cap for syringes, including means for capturing a needle which is introduced and forced therein so as to block extraction of the used needle from it, the cap being so shaped that it can be put and stand in upright position on a support plane, with its open end facing upward, thus permitting easy and simple introduction ; and blocking of the used needle into it without needing to hand hold the cap.

It is to be noted that, if the cap stands on a rigid surface, it is possible to apply a strong force on it with the syringe which is hand held by the operator, so that the needle can be easily forced to snap into the cap.

Consequently, the syringe, according to the invention comprises a needly having, at its end opposite to its tip, a collar with a frustum of cone shaped outer surface, a cap being provided to protect the needle after use, said cap having a cavity in which the needle can be completely inserted and protected, said cavity being open at one of its ends where a seat that is substantially complementary to that of the needle collar is formed, the cap, close to its open end, being provided with elastically deformable catching members, said members strongly engaging the collar of the needle when the needle is forced and completely pushed into the cap cavity, characterized in that said cap at its closed end is provided with a support base upon which the cap can stand in upright position on any substantially horizontal resistant surface.

The cap, after having been removed from the needle, can be put and stand in upright position on a support plane, so that the syringe needle, after the syringe has been used, can be easily and safely introduced into the open end of the cap and forced through it until the needle is safely blocked into the cap, without any risk that the person handling the syringe can puncture his fingers while applying the cap onto the needle.

The invention will be better understood based on the following description of ohe embodiment thereof, which is merely an illustrative example. In the description, reference is made to the enclosed drawing in which :

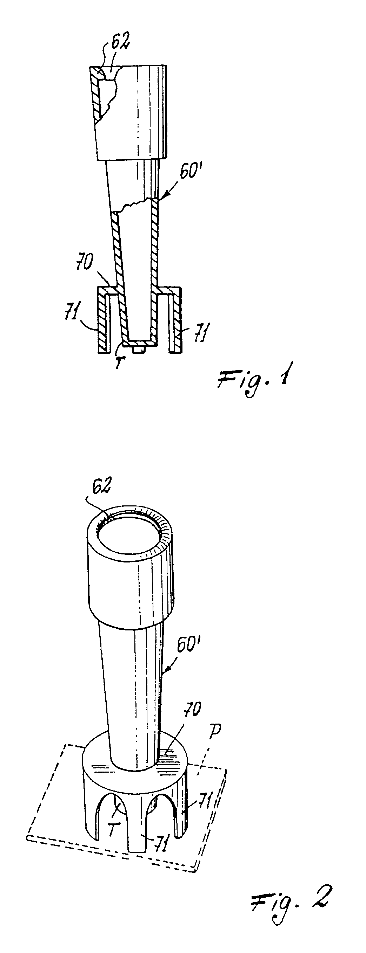

Figures 1 and 2 show a partly sectional side elevation and a perspective view, respectively, of an embodiment of the needle protection cap.

In figures 1 and 2, an embodiment 60' of the needle protection cap is shown, said needle protection cap being internally provided close to its opening, with an annular saw-tooth shaped rib 62 the steeper side of which is directed downward ; from the external surface of the cap, in an intermediate position thereof and especially near its closed end, there is an enlarged base consisting of an annular flange 70 from which appendixes or feet 71 protude.

At the moment of assembling the syringe, the machine that applies the cap 60' upon the needle will exert thereon an axial force not so strong as to make it difficult to remove the cap with the hands at the moment the syringe is to be used, without causing the needle to be removed from the syringe barrel.

Cap 60', after it has been removed from needle (to the purpose of allowing the syringe to be used) can be laid in a stable upright position onto a support plane P, a portion of which is shown in dashed lines for the sake of evidence in figure 2.

After the syringe has been used, cap 60' is clearly visible on its support plane and needle can be introduced into the seat of said cap without the need that it is held with the hands, as can be easily understood, thus avoiding the risk that the needle point may sting the operator's fingers.

After that, it will be enough to force the needle into the cap until rib 62, due to its resilient deformation, shaps beyond the upper edge of the collar of the needle, thus trapping it.

It is evident that also embodiments different from those shown in the figures can be suited to keep cap 60' in an upright position on a support plane : for example, the same end base T of the cap can be made larger so as to become a firm support base, or three or more support feet can protrude directly from the external surface of cap 60' towards its free end T.

## Claims

1. A syringe for medical use of the disposable type, comprising a needle having, at its end opposite to its tip, a collar with a frustum of cone shaped outer surface, a cap (60') being provided to protect the needle after use, said cap having a cavity in which the needle can be completely inserted and protected, said cavity being open at one of its ends where a seat that is substantially complementary to that of the needle collar is formed, the cap, close to its open end, being provided with elastically deformable catching members, said members strongly engaging the collar of the needle when the needle is forced and completely pushed into the cap cavity, characterized in that said cap at its closed end is provided with a support base upon which the cap can stand in upricht position on any substantially horizontal resistant surface.

2. A syringe according to claim 1, characterized in that said base comprises a flange (70), said flange projecting from the cap and having at least three feet or appendixes (71) protruding towards the closed end of the cap.

## Ansprüche

1. Spritze vom Wegwerf-Typ für medizinische Zwecke, mit einer Nadel, an deren der Spitze gegenüberliegendem Ende ein Ansatz mit kegelstumpfförmiger Außenfläche vorgesehen ist, sowie einer zum Schutz der Nadel nach deren Gebrauch vorgesehenen Kappe (60'), die einen Hohlraum aufweist, in welchen die Nadel vollständig einführbar ist und in welchem sie geschützt ist, wobei der Hohlraum an einem seiner Enden offen ist, an welchem ein im wesentlichen zum Nadelansatz komplementärer Sitz ausgebildet ist, wobei die Kappe nahe ihrem offenen Ende mit elastisch verformbaren Rastteilen versehen ist, die fest an dem Ansatz der Nadel angreifen, wenn die Nadel vollständig in den Hohlraum der Kappe gedrückt ist, dadurch gekennzeichnet, daß die Kappe an ihrem geschlossenen Ende mit einer Stützbasis versehen ist, auf der die Kappe auf jeglicher im wesentlichen horizontalen, unnachgiebigen Fläche aufrecht stehen kann.

2. Spritze nach Anspruch 1, dadurch gekennzeichnet, daß die Basis einen Flansch (70) aufweist, der von der Kappe absteht und wenigstens drei Füße oder Ansätze (71) aufweist, die zum geschlossenen Ende der Kappe hin vorstehen.

## Revendications

1. Seringue à usage médical, du type jetable, comprenant une aiguille ayant, à son extrémité opposée à sa pointe, un collier ayant une surface extérieure en forme de tronc de cône, un capuchon (60') étant prévu pour protéger l'aiguille après usage, ledit capuchon ayant une cavité dans laquelle l'aiguille peut être complètement introduite et protégée, ladite cavité étant ouverte à l'une de ses extrémités où un siège, qui est sensiblement complémentaire de celui du collier de l'aiguille, est formé, le capuchon, près de son extrémité ouverte, étant doté d'éléments d'accrochage, élastiquement déformables, lesdits éléments venant fermement en prise avec le collier de l'aiguille lorsque l'aiguille est enfoncée et poussée complètement dans la cavité du capuchon, caractérisée par le fait que ledit capuchon, à son extrémité fermée, est doté d'une base support sur laquelle le capuchon peut se tenir en position verticale sur n'importe quelle surface résistante sensiblement horizontale.

2. Seringue selon la revendication 1, caractérisée par le fait que ladite base comprend une collerette (70), ladite collerette saillant sur le capuchon et ayant au moins trois pieds ou appendices (71) en saillie vers l'extrémité fermée du capuchon.

*Fig. 1*

*Fig. 2*